# EUROPÄISCHE PATENTSCHRIFT

(11) **EP 1 261 288 B1**
(45) Veröffentlichungstag und Bekanntmachung des Hinweises auf die Patenterteilung: **14.06.2006**
(21) Anmeldenummer: 02715391.5
(22) Anmeldetag: 04.01.2002
(51) Int. Cl.: A61B 17/70

(54) **VERBINDUNGSELEMENT FÜR KNOCHEN- ODER WIRBELSTÄBE**
CONNECTOR ELEMENT FOR BONE RODS OR SPINAL RODS
ELEMENT DE CONNEXION POUR BARRES DE STABILISATION D'OS OU DE VERTEBRES

(30) Priorität: 12.01.2001 DE 10101478
(43) Veröffentlichungstag der Anmeldung: 04.12.2002
(73) Patentinhaber: BIEDERMANN MOTECH GmbH, 78054 VS-Schwenningen (DE)
(72) Erfinder: BIEDERMANN, Lutz, 78048 VS-Villingen (DE); HARMS, Jürgen, 76227 Karlsruhe (DE); OSTERMANN, Peter, 46397 Bocholt (DE)
(74) Vertreter: Hofer, Dorothea
(86) Internationale Anmeldenummer: PCT/EP2002/000042
(87) Internationale Veröffentlichungsnummer: WO 2002/054965

(56) Entgegenhaltungen:
- EP-A- 0 514 303
- EP-A- 0 732 081
- WO-A-00/59387
- FR-A- 2 795 622
- US-A- 5 980 523

## Beschreibung

Die Erfindung betrifft ein Verbindungselement nach dem Oberbegriff des Patentanspruches 1 zum Verbinden zweier zur Knochen- bzw. Wirbelstabilisierung eingesetzter stabförmiger Elemente.

Ein derartiges Verbindungselement ist aus der DE 692 06 318 T2 bekannt. Das Verbindungsteil ist aus zwei miteinander an einem Ende zu verbindenden Stäben gebildet, die an den zu verbindenden Elementen von Fassungen erfasst werden, die um eine gemeinsame sich quer zu der Stabrichtung erstreckenden Schraube schwenkbar ausgebildet sind. Die Enden des Verbindungsteiles weisen Ringe auf, die auf Schrauben aufsteckbar sind, welche mit den zu verbindenden Stäben verbindbar sind. Aus der WO 91/16020 ist ein Verbindungsteil bekannt, welches zwei zylinderabschnittsförmige Kanäle aufweist, die zur Aufnahme zweier zueinander paralleler Stäbe dienen. Eine Verbindung zueinander geneigter oder zueinander schräg verlaufender Stäbe ist damit nicht möglich.

Aus der FR 2795 622 ist ein Verbindungselement nach dem oberbegrifft des Patentanspruchs 1 bekannt.

Aufgabe der Erfindung ist es, ein Verbindungselement der eingangs beschriebenen Art zu schaffen, welches eine Verbindung von zueinander geneigten oder zueinander schief verlaufenden Stäbe in verbesender Weise ermöglicht.

Diese Aufgabe wird durch das in Patentanspruch 1 gekennzeichnete Verbindungselement gelöst. Ein solches Verbindungselement hat den großen Vorteil, daß der Chirurg in der Ausrichtung der Stäbe in Abhängigkeit von der gewünschten Ausrichtung der zu verbindenden Teile frei ist.

Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren. Von den Figuren zeigen:
Fig. 1. eine perspektivische Darstellung eines Anwendungsfalles;
Fig. 2 eine Schnittdarstellung durch eine erste Ausführungsform eines Verbindungselements; und
Fig. 3 eine Schnittdarstellung durch eine zweite Ausführungsform eines Verbindungselements; und
Fig. 4 eine Schnittdarstellung durch eine abgewandelte Ausführungsform.

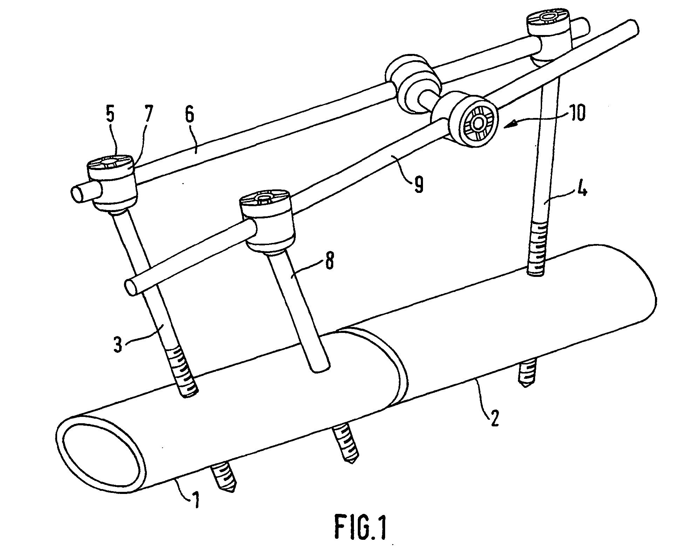

In Fig. 1 sind zwei miteinander zu verbindende und gegeneinander zu fixierende Röhrenknochenteile 1 gezeigt. In jedes der beiden Teile ist eine aus der Wirbelsäulenchirurgie als Pedikelschraube bekannte Schraube 3, 4 eingeschraubt. Jede der Schrauben weist einen Kopf 5 mit einer Aufnahmeöffnung zum Aufnehmen eines Stabes 6 auf. Mit Hilfe einer Mutter 7 ist der Stab mit dem Kopf fest verbunden. Wie aus Fig. 1 ersichtlich ist, ist in dem Teil 1 eine dritte Schraube 8 eingeschraubt, die genau wie die ersten beiden Schrauben ausgebildet ist und einen zweiten Stab 9 in ihrem Kopf aufnimmt.

Der zweite Stab 9 ist über ein Verbindungselement 10 mit dem ersten Stab 6 verbunden. Dadurch wird eine zusätzliche Stabilisierung eingerichtet.

In dem obigen Ausführungsbeispiel sind die beiden zu verbindenden Teile 1 und 2 Röhrenknochenteile. In gleicher Weise erfolgt eine Anwendung von wenigstens 2 Stäben mit entsprechenden Schrauben zur Aufnahme der selben bei der Wirbelsäulenchirurgie, etwa bei einem Fixateur intern, wie er beispielsweise aus der DE 38 23 737-C bekannt ist.

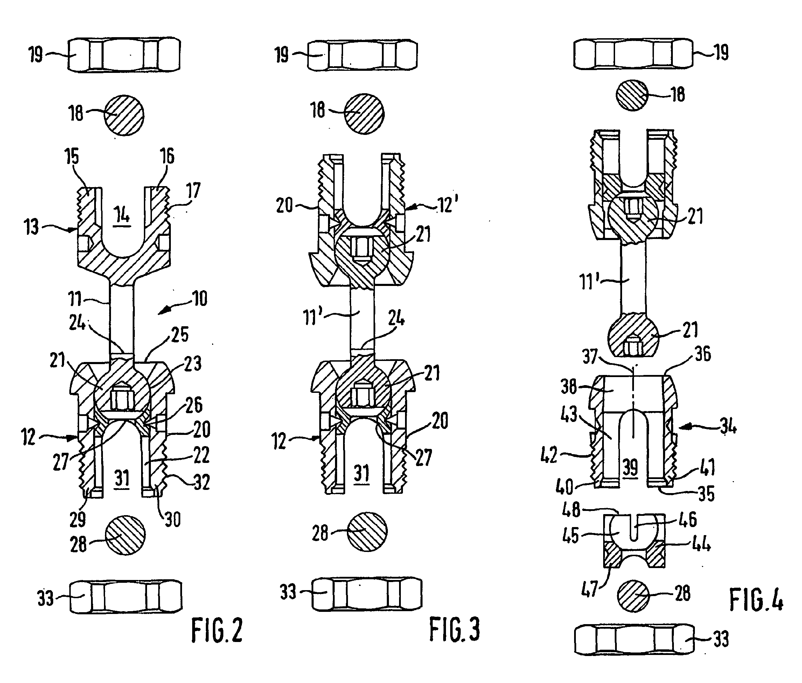

Das Verbindungselement 10 weist in der in Fig. 2 gezeigten Ausführungsform einen Schaft 11 mit einem ersten Teil 12 an seinem einen Ende und einem zweiten Teil 13 an seinem anderen Ende auf.

Das zweite Teil 13 umfaßt zwei einen U-förmigen Kanal 14 begrenzende Schenkel 15, 16. Die beiden Schenkel 15, 16 weisen außen eine zylindrische Form auf und besitzen an ihren freien Enden ein Außengewinde 17. Der U-förmige Kanal 14 weist einen Durchmesser auf, der im wesentlichen gleich oder wenig größer als der Durchmesser eines aufzunehmenden Stabes 18 ist und der gerade so groß gewählt ist, daß der Stab 18 in den U-förmigen Kanal 14 einführbar und von diesem in seitlicher Richtung geführt ist. Ferner ist eine Mutter 19 vorgesehen, die mit ihrem Innengewinde so ausgestattet ist, daß sie mit dem Außengewinde 17 zusammen wirkt. Das Gewinde 17 ist so ausgebildet, daß der Abstand von dem Gewinde zum Grund des U-förmigen Kanals 14 kleiner ist, als der Durchmesser des Stabes 18, so daß der in den U-förmigen Kanal 14 eingelegte Stab 18 durch Aufschrauben der Mutter 19 fixierbar ist.

Wie aus Fig. 2 ersichtlich ist, weist das andere Ende des Schoftes 11 einen einen sphärischen Abschnitt aufweisenden Kopf 21 auf. Das erste Teil 12 weist eine zylindrisch ausgebildete Fassung 20 auf, deren Durchmesser im wesentlichen gleich bzw. ein wenig größer als der Durchmesser des Kopfes 21 ist und so bemessen ist, daß der Kopf in der Bohrung einführbar und von dieser geführt ist.

In dem gezeigten Ausführungsbeispiel weist die Bohrung 22 einen Grund 23 auf, der sphärisch ausgebildet ist, wobei sein Radius gleich dem Radius des Kopfes 21 ist. Wie aus den Figuren 2 und 3 ersichtlich ist, ist der Schaft 11 über eine Schraubverbindung von dem kugelförmigen Kopf abtrennbar ausgebildet.

In einer Ausführungsform ist das Verbindungselement 10 so ausgebildet, daß eine Trennung von dem ersten und zweiten Teil möglich ist. In dem in den Figuren 2 und 3 gezeigten Ausführungbeispiel erfolgt die Trennung an der mit 24 bezeichneten Stelle des Schaftes 11 bzw. 11'. Zu diesem Zweck weist ein Schaftteil eine Gewindebohrung auf, während der andere Schaftteil einen Gewindeansatz aufweist, der in die Gewindebohrung einschraubbar ist. Diese Verbindung ist schematisch durch die Verbindungsstelle 24 angedeutet. Die Gewindebohrung kann auch im Kopf 21 direkt vorgesehen sein, und der Schaft 11 hat dann an seinem dem Kopf zugewandten Ende einen entsprechend ausgebildeten Schraubansatz, der mit der Gewindebohrung zum Zusammenschrauben der beiden Teile in Eingriff bringbar ist.

In der in Fig. 2 gezeigten Ausführungsform ist zunächst der Schaft 11 von dem Kopf 21 abgeschraubt, so daß der Kopf 21 von dem freien Ende der Bohrung 22 in diese in die gezeigte Stellung einführbar ist. Es wird dann der Schaft 11 angeschraubt, so daß die in der Figur gezeigte Verbindung hergestellt ist.

Wie Fig. 2 ferner erkennen läßt, grenzt an den Grund 23 eine zweite Bohrung 25 an, die nach außen trichterförmig ausgeweitet ist, so daß der Schaft 11 um einen Raumwinkel relativ zur Fassung 20 schwenkbar ist.

Nach dem eingeführten Kopf 21 wird in die Bohrung 22 ein Druckelement 26 eingesetzt, welches auf seiner dem Kopf zugewandten Seite sphärisch ausgebildet ist mit einem Radius, der dem Radius des Kopfes 21 entspricht. Auf seiner dem Kopf 21 abgewandten Seite weist das Druckelement eine zylindrische Oberfläche 27 auf, bei der der Durchmesser dieses Zylinders nahezu gleich dem Durchmesser des aufzunehmenden Stabes 28 ist, derart daß der Stab 28 in die Ausnehmung 27 einlegbar ist. Wie aus Fig. 2 ersichtlich ist, ist die Ausnehmung ferner so ausgebildet, daß ein zu der Bohrung 22 koaxialer, U-förmiger Kanal 31 mit diesen begrenzenden Schenkeln 29, 30 gebildet ist. Die beiden Schenkel 29, 30 weisen wie das zweite Teil 13 angrenzend an ihre freien Enden ein Außengewinde 32 auf, welches sich soweit zu dem anderen Ende der Fassung 20 hin erstreckt, daß sein Abstand von dem Boden der zylindrischen Ausnehmung kleiner ist als der Durchmesser des Stabes 28. Ferner ist eine Mutter 33 vorgesehen, deren Innengewinde dem Außengewinde 32 entspricht.

Im Betrieb wird in dem wie oben beschrieben vorbereiteten Verbindungsteil zunächst der Stab 18 in das monoaxial wirkende zweite Teil 13 bzw. in dessen U-förmigen Kanal 14 eingesetzt. Dann wird der Stab 18 mit Hilfe der Mutter 19 fixiert. Anschließend wird die Fassung 20 so ausgerichtet, daß sie den zweiten Stab 28 in sich aufnimmt. Nach Einführen des Zwischenstabes 28 wird die Mutter 33 aufgeschraubt. Diese fixiert nicht nur den Stab 28, sondern übt über den Stab 28 und das Druckelement 26 auch einen solchen Druck auf den Kopf 21 auf, daß dieser in seiner Achslage fixiert wird.

Bei der in Fig. 3 gezeigten Ausführungsform stimmen die Fassung 20, das Druckelement 26, der Kopf 21, der Stab 28 und die Mutter 33 in allen Einzelheiten mit dem ersten Teil 12 in Fig. 2 überein. Die Ausführungsform unterscheidet sich lediglich dadurch, daß das zweite Teil nicht als monoaxiale Verbindung sondern als eine ebenfalls polyaxiale Verbindung ausgebildet ist. Dieses zweite Teil 12' ist zu dem ersten Teil 12 spiegelsymetrisch ausgebildet. Alle Teile von Fassung, Kopf, Druckelement, Stab und Mutter stimmen mit den entsprechenden Teilen des ersten Teiles 12 überein. Zur Montage ist der Schaft 11' wie in dem vorher beschriebenen Ausführungsbeispiel durch eine Schraubverbindung entlang der Linie 24 lösbar. Nach Einsetzen der Köpfe 21 werden die beiden Teile des Schaftes 11' fest miteinander verbunden.

Im Betrieb erfolgt die Verbindung wie vorher anhand des ersten Teiles 12 beschrieben, wobei jede der Fassungen 20 vor dem Fixieren jeweils auf den aufzunehmenden Stab 18, 28 hin orientiert wird und dann der Stab eingelegt und über die Mutter 19, 33 fixiert wird. Durch Ausüben des Druckes von der Mutter über den Stab 18, 28 und das Druckelement wird dann auch der Kopf endgültig in seiner Lage fixiert.

In einer nicht gezeigten abgewandelten Ausführungsform kann die Fassung 20 bzw. das zweite Teil 13 jeweils an seinem freien Ende angrenzend noch ein Innengewinde aufweisen, in welches eine Innenschraube einschraubbar ist, um auf diese Weise in an sich bekannter Art eine Blockierung der Verschraubung zu erreichen.

Bei den oben beschriebenen Ausführungsformen ist der Kopf 21 jeweils vom freien Ende der U-förmigen Ausnehmung her eingesetzt. In einer abgewandelten Ausführungsform kann die Bohrung 22 sich durch die gesamte Fassung erstrecken. Der Kopf wird dann von dem den Schenkeln abgewandten Ende der Bohrung 22 her eingesetzt und mit einem anzubringenden Sperring oder Sprengring in der Bohrung gehalten. Entscheidend ist, daß der freie Rand, der an dem dem Schaft 11 zugewandten Ende den Kopf umgibt, nach außen kegelförmig aufgeweitet ist, um eine polyaxiale Bewegung zwischen Fassung und Kopf bzw. Schaft zu ermöglichen.

Die in Fig. 4 gezeigte Ausführungsform stimmt bezüglich Schaft 11', Kopf 21, Stab 18 bzw. 28 und Mutter 19 bzw. 33 vollständig mit der in Fig. 3 beschriebenen Ausführungsform überein. Anders ausgebildet ist die Fassung 34. Diese weist ein erstes Ende 35 und ein diesem gegenüberliegendes zweites Ende 36 auf. Es ist eine zur Symmetrieachse 37 der Fassung koaxiale Bohrung 38 vorgesehen. Ferner ist eine von dem ersten Ende 35 ausgehende U-förmige Ausnehmung 39 vorhanden, die sich quer zur Symmetrieachse 39 erstreckt. Die U-förmige Ausnehmung wird von zwei Schenkeln 40, 41 begrenzt. Angrenzend an das erste Ende 35 weisen die beiden Schenkel 40, 41 ein Außengewinde 42 auf. Der Durchmesser der U-förmigen Ausnehmung 39 ist gleich bzw. ein wenig größer als der Durchmesser des Stabes 18 bzw. 28 und gerade so groß, daß der Stab in die Ausnehmung einführbar ist und von den Seiten der U-förmigen Ausnehmung seitlich geführt ist. Das Außengewinde 42 ist wie bei den zuvor beschriebenen Ausführungformen so weit zum Grund der U-förmigen Ausnehmung ausgebildet, daß der Abstand zum U-förmigen Grund kleiner ist als der Durchmesser des aufzunehmenden Stabes. Ferner ist wie bei dem zuvor beschriebenen Ausführungsbeispiel eine Mutter 33 vorgesehen, die mit dem Außengewinde 42 zusammenwirkt.

Anders als in den zuvor beschriebenen Ausführungsbeispielen erstreckt sich der zylindrische Abschnitt 43 der koaxialen Bohrung bis zu einem vorgegebenen Abstand zum zweiten Ende 36 und ist von da ab zum zweiten Ende 36 hin mit einem Kegelwinkel konisch verjüngt. Ferner ist ein Druckelement 44 vorgesehen, dessen Außenfläche in einem den Kopf 21 seitlich umfassenden Bereich 45 gegen das zweite Ende 36 hin kegelig ausgebildet ist, wobei der Kegelwinkel dem des kegeligen Bereiches der Bohrung entspricht. Der kegelige Bereich weist eine zum zweiten Ende 36 hin gerichtete und zu diesem Ende hin offen verlaufende Schlitzung 46 auf. Durch die Anpassung der Kegelflächen zwischen Bohrung 38 und Druckelement 44 folgt bei vollständig eingeschobenem Zustand eine Selbsthemmung. Das Druckelement weist in der aus der EP 0 732 081 B bekannten Weise ein erstes Ende 47 und ein diesem gegenüberliegendes zweites Ende 48 auf. Angrenzend an das erste Ende ist ein im wesentlichen zylinderförmiger Abschnitt vorgesehen, dessen Außendurchmesser so gewählt ist, daß das Druckelement in dem zylindrischen Abschnitt 43 gleiten kann. Wie aus der Figur ersichtlich ist, weist das Druckelement in seinem zweiten Bereich eine zu dem zweiten Ende 48 hin offene kugelsegmentförmige Ausnehmung 45 zur Aufnahme des Schraubenkopfes auf. Im übrigen stimmen die Fassung 34 und das Druckelement 44 mit der Offenbarung in der genannten EP 0 732 081 überein.

Die in Fig. 4 gezeigte Einrichtung ist spiegelsymmetrisch ausgebildet, so daß das andere Ende des Schaftes 11' mit seinem Kopf, der Fassung und dem Druckelement identisch mit dem zuvor beschriebenen ausgebildet sind.

Im Betrieb wird das Druckelement von dem ersten Ende 35 her in die Fassung 34 eingeschoben. Von dem zweiten Ende 36 her wird der Kopf 21 in den Bereich 45 eingeschoben bzw. hineingedrückt. Dann wird der Stab 28 in den verbleibenden U-förmigen Schlitz eingelegt, und über das Aufschrauben der Außenmutter 33 wird Druck auf den Stab 28 und über diesen auf das den Kopf 21 umfassende Druckelement 44 so ausgeübt, daß Stab 28 und Kugelkopf 21 in ihrer Lage fixiert werden. Entsprechend wird auf der gegenüberliegenden Seite vorgegangen.

Auch diese Version läßt eine Schwenkung des Schaftes 11' um einen vorgegebenen Raumwinkel und die Symmetrieachse 37 zu, so daß eine Ausrichtung bzw. Anpassung auf die zu verbindenden Stäbe bzw. Stäbe mit Schrauben möglich wird.

Alternativ kann die Vorrichtung auch so ausgebildet sein, daß ein Kopf mit Fassung in der in Fig. 4 gezeigten Weise ausgebildet ist und der andere Kopf in der in Fig. 2 beschriebenen monoaxialen Weise geformt ist.

Bei den oben beschriebenen Ausführungsbeispielen sind die Verbindungselemente stets zur Verbindung von Stäben erläutert. In gleicher Weise können auch zwei Schäfte von zwei Schrauben oder ein Schaft einer Schraube und ein Stab mit dem Verbindungselement verbunden und fixiert werden.

## Patentansprüche

1. Verbindungselement (10) zum Verbinden zweier zur Knochen- bzw. Wirbelstabilisierung eingesetzter stabförmiger Elemente (6, 9, 18, 28, 3, 4, 8) mit einem ein erstes Ende und ein gegenüberliegendes zweites Ende aufweisenden stabförmigen Verbindungsteil (11, 11') und je einem Endteil (12, 13, 12') an seinen beiden Enden, von denen wenigstens eines eine polyaxiale Ausrichtung zwischen stabförmigem Element und Verbindungsteil erlaubt, **dadurch gekennzeichnet, dass** das eine polyaxiale Ausrichtung erlaubende Endteil (12, 13, 12') zwei einen U-förmigen Kanal (14) begrenzende Schenkel (15, 16) zum Aufnehmen des stabförmigen Elements (28), ein an den Schenkeln vorgesehenes Innen- bzw. Außen-Gewinde (17) und eine mit dem Gewinde zusammenwirkende Mutter bzw. Schraube (19) zum Fixieren des eingelegten Stabes (28) in dem Kanal aufweist.

2. Verbindungselement nach Anspruch 1, **dadurch gekennzeichnet, daß** das zweite Endteil (13) mit dem Verbindungsteil (10) monoaxial verbunden ist.

3. Verbindungselement nach Anspruch 1, **dadurch gekennzeichnet, daß** beide Endteile (12, 12') polyaxial ausrichtbar verbunden sind.

4. Verbindungselement nach einem der Ansprüche 1 bis 3, **dadurch gekennzeichnet, dass** das Verbindungsteil (11, 11') an seinem dem stabförmigen Element (28) zugewandten Ende einen Kopf (21) und das die polyaxiale Ausrichtung ermöglichende Endteil (12, 12') ein Aufnahmeelement (20) mit einer Aufnahmeöffnung (31, 22) zum Aufnehmen des stabförmigen Elementes (28) einerseits und des Kopfes (21) andererseits aufweist.

## Claims

1. Connecting element (10) for connecting two rod-shaped elements (6, 9, 18, 28, 3, 4, 8) which are used for bone or vertebra stabilisation,
with a rod-shaped connecting portion (11, 11') comprising a first end and an opposing second end, and
with a respective end portion (12, 13, 12') on both of its ends of which at least one allows a polyaxial orientation between the rod-shaped element and the connecting portion,
**characterised in that**
the end portion (12, 13, 12') allowing polyaxial orientation comprises two legs (15, 16) defining a U-shaped channel (14) for receiving the rod-shaped element (28), an inner thread or outer thread (17) provided on the legs, and a nut or screw (19) cooperating with the thread for fixing the inserted rod (28) in the channel.

2. Connecting element according to claim 1, **characterised in that** the second end portion (13) is monoaxially connected to the connecting portion (10).

3. Connecting element according to claim 1, **characterised in that** both end portions (12, 12') are connected so that they can be oriented polyaxially.

4. Connecting element according to any one of claims 1 to 3, **characterised in that** the connecting portion (11, 11') comprises a head (21) at its end facing towards the rod-shaped element (28) and the end portion (12, 12') allowing the polyaxial orientation comprises a receiving element (20) with a receiving opening (31, 22) for receiving the rod-shaped element (28) on the one hand and the head (21) on the other hand.

## Revendications

1. Elément de connexion (10) pour la connexion de deux éléments (6, 9, 18, 28, 3, 4, 8) en forme de barres, utilisés pour la stabilisation des eaux ou de la colonne vertébrale, avec une partie de connexion (11,11') en forme de barre, présentant une première extrémité et une deuxième extrémité opposée et, respectivement, une partie d'extrémité (12, 13, 12') à ses deux extrémités, dont au moins l'une permet une orientation polyaxiale, entre un élément en forme de barre et une partie de connexion, **caractérisé en ce que** la partie d'extrémité (12, 13, 12'), permettant une orientation polyaxiale, présente deux branches (15, 16) délimitant un canal (14) en forme de U pour loger l'élément (28) en forme de barre, un filetage intérieur ou extérieur (17), prévu sur les branches, et un écrou ou vis (19) coopérant avec le filetage, pour la fixation de la barre (28), insérée, dans le canal.

2. Elément de connexion selon la revendication 1, **caractérisé en ce que** la deuxième partie d'extrémité (13) est reliée de manière monoaxiale à la partie de liaison (10).

3. Elément de connexion selon la revendication 1, **caractérisé en ce que** les deux parties d'extrémité (12, 12') sont reliées de façon à pouvoir être orientée de façon polyaxiale.

4. Elément de connexion selon l'une des revendications 1 à 3, **caractérisé en ce que** la partie de liaison (11, 11') présente, sur son extrémité, tournée vers l'élément (28) en forme de barre, une tête (21), et la partie d'extrémité (12, 12') permettant l'orientation polyaxiale présente un élément de logement (20) ayant une ouverture de logement (31, 22), pour recevoir l'élément (28) en forme de barre, d'une part, et la tête (21), d'autre part.
